(19) Europäisches Patentamt
European Patent Office
Office européen des brevets

(11) Veröffentlichungsnummer: **0 014 409**
**B1**

(12) # EUROPÄISCHE PATENTSCHRIFT

(45) Veröffentlichungstag der Patentschrift: **12.05.82**

(21) Anmeldenummer: **80100427.6**

(22) Anmeldetag: **28.01.80**

(51) Int. Cl.³: **C 07 C 102/00,** C 07 C 103/34, C 07 D 209/08, C 07 D 215/08, C 07 D 211/16, C 07 D 295/18

(54) **Verfahren zur Herstellung von Alpha-Hydroxycarbonsäureamiden.**

(30) Priorität: **07.02.79 DE 2904490**

(43) Veröffentlichungstag der Anmeldung: **20.08.80 Patentblatt 80/17**

(45) Bekanntmachung des Hinweises auf die Patenterteilung: **12.05.82 Patentblatt 82/19**

(84) Benannte Vertragsstaaten: **BE CH DE FR GB IT NL**

(56) Entgegenhaltungen:
**DE-A-2 201 432**
**DE-A-2 219 923**
**US-A-3 399 988**
**E. MÜLLER: »Methoden der Organischen Chemie«, Houben Weyl, 4. Auflage, Band VIII, 1952 Georg Thieme Verlag, Stuttgart, DE, »Sauerstoffverbindungen III«, Seiten 526—527**
**C. M. STARKS & C. LIOTTA: »Phase transfer catalysis. Principles and techniques«, 1978, Academic Press, US, Seiten 140—155**

(73) Patentinhaber: **BAYER AG, Zentralbereich Patente, Marken und Lizenzen, D-5090 Leverkusen 1, Bayerwerk (DE)**

(72) Erfinder: **Diehr, Hans-Joachim, Dr., Höhe 35, D-5600 Wuppertal 11 (DE)**

Anmerkung. Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 014 409 B1

BUNDESDRUCKEREI BERLIN

## Verfahren zur Herstellung von α-Hydroxycarbonsäureamiden

Die Erfindung betrifft ein neues Verfahren zur Herstellung von teilweise bekannten α-Hydroxycarbonsäureamiden, welche als Zwischenprodukte zur Herstellung von Herbiziden verwendet werden können.

Es ist bereits bekannt geworden, daß man Glycolsäureanilide erhält, wenn man Chloressigsäureanilide mit Acetat zu Acetoxyessigsäureaniliden umsetzt und letztere alkalisch verseift (vergleiche DE-OS 2 201 432).

Zur Herstellung der Acetoxyessigsäureanilide werden beispielsweise nach dem Stand der Technik die entsprechenden Chloressigsäureanilide mit einem hohen Überschuß an Kaliumacetat in verdünnter Essigsäure 20 Stunden unter Rückfluß erhitzt.

Nach dem Erkalten können dann im allgemeinen die entstandenen Acetoxyessigsäureanilide durch Filtration isoliert werden. Als Nachteile dieses Verfahrens sind der hohe Aufwand an Kaliumacetat sowie die Belastung der Mutterlauge mit dem überschüssigen Acetat und dem als Koppelprodukt gebildeten Kaliumchlorid anzusehen.

Es ist ferner bekannt, daß die hier interessierende, schon lange bekannte Esterbildungsreaktion in einem flüssigen Zweiphasensystem — nämlich die Umsetzung von wäßrigen Lösungen von Carbonsäure-alkalisalzen mit in einer zweiten flüssigen, nicht-wäßrigen Phase gelösten Alkalihalogeniden — durch bestimmte in der wäßrigen Phase gelöste quartäre Ammoniumsalze, welche hierbei als Phasentransfer-Katalysatoren fungieren, katalysiert werden kann. Hierbei liegen alle Reaktionsteilnehmer, d. h. Ausgangs- und Endprodukte, sowie der Phasentransfer-Katalysator in gelöster Form vor (vgl. z. B. C. M. Starks and C. Liotta, »Phase Transfer Catalysis. Principles and Techniques«, 1978, Academic Press, U. S., Seiten 140—155).

Es wurde nun gefunden, daß man α-Hydroxycarbonsäureamide der Formel

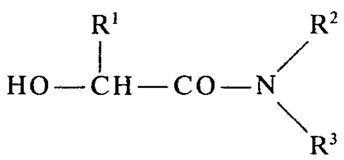

(I)

in welcher

$R^1$ für Wasserstoff oder Alkyl steht,

$R^2$ und $R^3$ gleich oder verschieden sind und einzeln für Wasserstoff, jeweils gegebenenfalls durch Cyano-, Alkoxy- oder Alkylthioreste substituiertes Alkyl, Alkenyl, Alkinyl oder Cycloalkyl, jeweils gegebenenfalls durch Halogenatome, Alkyl-, Nitro- und/oder Cyanoreste substituiertes Aralkyl oder Aryl oder einen stickstoffhaltigen heterocyclischen Rest stehen oder zusammen mit dem Stickstoffatom, an das sie gebunden sind, einen gegebenenfalls durch Alkylgruppen substituierten, gegebenenfalls teilweise ungesättigten und gegebenenfalls benzanellierten Mono- oder Bicyclus bilden, der gegebenenfalls weitere Heteroatome enthält,

durch Umsetzung von α-Halogencarbonsäureamiden mit Acetaten zu α-Acetoxycarbonsäureamiden und Entacylierung dieser Zwischenprodukte, dadurch gekennzeichnet, daß man in erster Stufe α-Halogencarbonsäureamide der Formel

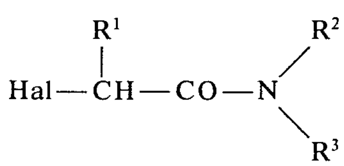

(II)

in welcher

$R^1$, $R^2$ und $R^3$ die oben angegebene Bedeutung haben und
Hal für Chlor oder Brom steht,

in Gegenwart eines quartären Ammoniumsalzes mit einem in einem inerten organischen Solvens suspendierten Alkali- oder Erdalkaliacetat bei Temperaturen zwischen 20 und 200°C umsetzt und in zweiter Stufe die auf diese Weise hergestellten α-Acetoxycarbonsäureamide der Formel

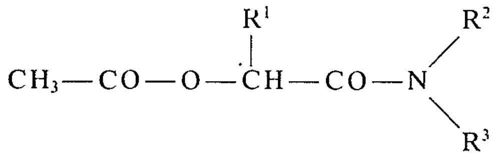

(III)

in welcher

$R^1$, $R^2$ und $R^3$ die oben angegebene Bedeutung haben,

in an sich bekannter Weise durch Umsetzung mit einem Alkohol der Formel

$$R^4{-}OH \qquad (IV)$$

in welcher

$R^4$ für Alkyl steht,

in Gegenwart katalytischer Mengen eines Alkali- oder Erdalkalihydroxides oder eines Alkali- oder Erdalkalicarbonates bei Temperaturen zwischen 20 und 150°C entacyliert.

Es ist als sehr überraschend zu bezeichnen, daß auch bei der Umsetzung von $\alpha$-Halogencarbonsäureamiden mit Alkali- bzw. Erdalkaliacetaten nach Stufe 1 des erfindungsgemäßen Verfahrens — Suspension der Ausgangsstoffe und des Katalysators in einem inerten Verdünnungsmittel — bestimmte quartäre Ammoniumsalze als Katalysatoren wirksam sind und zu nahezu quantitativen Ausbeuten führen, da hier nicht wie bei vorbekannten Verfahren ein flüssiges wäßriges Zweiphasensystem als Reaktionsmedium vorliegt (wobei alle Reaktionsteilnehmer, entweder in der wäßrigen oder in der nicht-wäßrigen Phase, gelöst sind), sondern nur eine flüssige Phase und mehrere feste (kristalline) Phasen; auch der Katalysator liegt in ungelöster, fester Form vor. Während die quartären Ammoniumsalze in dem wäßrigen Zweiphasensystem bekanntermaßen als sogenannte Phasentransferkatalysatoren fungieren, ist ihre Wirkungsweise im Falle des erfindungsgemäßen Verfahrens noch ganz unbekannt. Wie Vergleichsversuche gezeigt haben, erreicht man ohne Katalysatorzusatz nur Ausbeuten von $<25\%$ der Theorie.

Vorteile des erfindungsgemäßen Verfahrens sind neben seiner einfachen Durchführbarkeit die Verwendung der Reagentien — soweit sie nicht gleichzeitig als Verdünnungsmittel dienen — in stöchiometrischer Menge, der einheitliche Reaktionsverlauf und die einfache Aufarbeitung. Eine Belastung der Umwelt mit Abfallstoffen wird dabei weitgehend vermieden, da Koppelprodukte und Lösungsmittel leicht abgetrennt und in reiner Form isoliert werden können.

Verwendet man als Ausgangsverbindung beispielsweise Chloressigsäuremorpholid und in der ersten Stufe Natriumacetat, in der zweiten Stufe Methanol als Reagentien, so kann der Reaktionsablauf durch folgendes Formelschema skizziert werden:

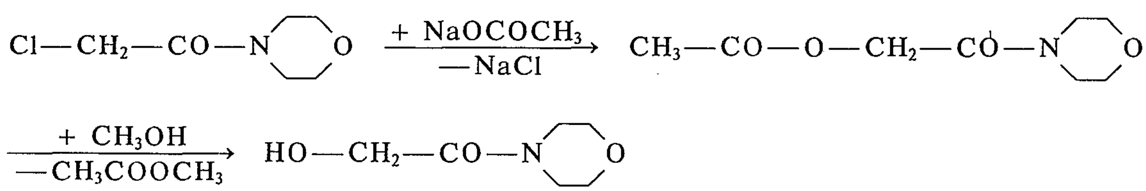


Die als Ausgangsverbindungen zu verwendenden $\alpha$-Halogencarbonsäureamide sind durch Formel (II) definiert.

Vorzugsweise stehen darin

$R^1$ für Wasserstoff oder Methyl,

$R^2$ und $R^3$, welche gleich oder verschieden sein können, einzeln für Wasserstoff, geradkettiges oder verzweigtes Alkyl mit 1 bis 20 Kohlenstoffatomen, Cyanoalkyl mit 2 bis 5 Kohlenstoffatomen, Alkoxyalkyl mit 2 bis 8 Kohlenstoffatomen, Alkylthioalkyl mit 2 bis 8 Kohlenstoffatomen, Alkenyl mit 3 bis 10 Kohlenstoffatomen, Alkinyl mit 3 bis 10 Kohlenstoffatomen, Cycloalkyl mit 3 bis 12 Kohlenstoffatomen, Aralkyl mit 1 bis 2 Kohlenstoffatomen im Alkylteil und 6 bzw. 10 Kohlenstoffatomen im Arylteil oder Aryl mit 6 bzw. 10 Kohlenstoffatomen stehen, wobei die beiden letztgenannten Reste substituiert sein können durch 1 bis 3 Halogenatome, insbesondere Fluor, Chlor und/oder Brom, 1 bis 3 Alkylreste mit jeweils 1 bis 4 Kohlenstoffatomen, Nitro und/oder Cyano;

$R^2$ und $R^3$ stehen darin vorzugsweise ferner für Morpholinyl oder Tetrahydrofurfuryl oder bilden zusammen mit dem Stickstoffatom, an das sie gebunden sind, einen gegebenenfalls durch 1 bis 3 Alkylgruppen mit jeweils 1 bis 5 Kohlenstoffatomen substituierten (wobei auch spiro-verknüpfte

Substituenten mit bis zu 4 Kohlenstoffatomen und bis zu 2 Sauerstoffatomen möglich sind), gegebenenfalls teilweise ungesättigten und/oder gegebenenfalls benzanellierten Monocyclus oder Bicyclus mit 3 bis 15 Kohlenstoffatomen, oder einen gegebenenfalls substituierten gesättigten und ein weiteres Stickstoffatom, ein Sauerstoffatom oder ein Schwefelatom enthaltenden Monocyclus von jeweils 3 bis 10 Kohlenstoffatomen, und

Hal steht vorzugsweise für Chlor.

Als Ausgangsstoffe sind besonders bevorzugt diejenigen Verbindungen der Formel (II), worin

$R^1$ für Wasserstoff steht,

$R^2$ und $R^3$, welche gleich oder verschieden sein können, einzeln für geradkettiges oder verzweigtes Alkyl mit 1 bis 10 Kohlenstoffatomen, Cyanoäthyl, 2-Alkoxyäthyl mit 1 bis 5, insbesondere 1 bis 3 Kohlenstoffatomen je Alkoxygruppe, Allyl, Propargyl, 1-Methylpropargyl, 1,1-Dimethylpropargyl, Cyclopentyl, Cyclohexyl, Phenyl, Nitrophenyl, Tolyl, Nitrotolyl, Chlorphenyl, Naphthyl, Benzyl, Chlorbenzyl, Chlortolyl, Morpholinyl oder den Tetrahydrofurfurylrest stehen oder zusammen mit dem Stickstoffatom, an das sie gebunden sind, für Pyrrolidyl, Monoalkyl- oder Dialkylpyrrolidyl mit 1 bis 5, insbesondere 1 bis 3 Kohlenstoffatomen je Alkylgruppe, Morpholinyl, Monoalkyl- oder Dialkylmorpholinyl mit 1 bis 5, insbesondere 1 bis 3 Kohlenstoffatomen je Alkylgruppe, Piperidyl, Monoalkyl-, Dialkyl- und Trialkyl-piperidyl mit jeweils 1 bis 5, insbesondere 1 bis 3 Kohlenstoffatomen je Alkylgruppe, für spirosubstituiertes Piperidyl, insbesondere den Rest der Formel

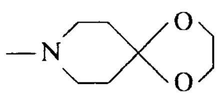

für Perhydro-azepinyl (=Hexamethylenimino-Rest), 1,2,3,4-Tetrahydroindolyl, Monoalkyl-, Dialkyl- und Trialkyl-1,2,3,4-tetrahydroindolyl mit jeweils 1 bis 5, insbesondere 1 bis 3 Kohlenstoffatomen je Alkylgruppe, Perhydroindolyl, Monoalkyl-, Dialkyl- und Trialkylperhydroindolyl mit jeweils 1 bis 5, insbesondere 1 bis 3 Kohlenstoffatomen je Alkylgruppe, 1,2,3,4-Tetrahydro-chinolyl und 1,2,3,4-Tetrahydroiso-chinolyl, Monoalkyl-, Dialkyl- und Trialkyl-1,2,3,4-tetrahydro-chinolyl und -isochinolyl mit jeweils 1 bis 5, insbesondere 1 bis 3 Kohlenstoffatomen je Alkylgruppe, Perhydrochinolyl und Perhydroisochinolyl, Monoalkyl-, Dialkyl- und Trialkylperhydro-chinolyl und -isochinolyl mit jeweils 1 bis 5, insbesondere 1 bis 3 Kohlenstoffatomen je Alkylgruppe, für Perhydrothiazolyl oder den Rest der Formel

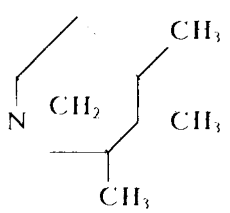

stehen und

Hal für Chlor steht.

Als Beispiele für die Halogencarbonsäureamide der Formel (II) seien genannt:

Chloressigsäure-methylamid, -äthylamid, n-propylamid, -iso-propylamid, -n-butylamid, iso-butylamid, -dimethylamid, -diäthylamid, -di-n-propylamid, -di-isopropylamid, -N-methyl-N-iso-propylamid, -N-methyl-N-iso-butylamid, -N-methyl-N-sek.-butylamid, -di-(2-äthylhexyl)-amid, N-methyl-N-(2-cyano-äthyl)-amid, -di-(2-methoxy-äthyl)-amid, -diallylamid, -N-methyl-N-propargyl-amid, -N-methyl-N-(1-methyl-propargyl)-amid, -dipropargylamid, -cyclopentylamid, -N-methyl-N-cyclopentylamid, -cyclo-hexylamid, -N-methyl-N-cyclohexylamid, -anilid, -2-nitro-, -3-nitro- und -4-nitro-phenylamid, -2-chlor-, -3-chlor- und -4-chlorphenylamid, -2,4-dichlor-, 2,5-dichlor-, -3,4-dichlor- und 3,5-dichlor-phenylamid, -2-methyl-, -3-methyl- und -4-methyl-phenylamid, -N-methyl-anilid, -N-methyl-N-(2-methyl-phenyl)-amid, -N-methyl-N-(2-nitrophenyl)-, -N-methyl-N-(3-nitrophenyl)-, -N-methyl-N-(4-nitrophenyl)-amid, -N-methyl-N-(2-chlorphenyl)-, -N-methyl-N-(3-chlorphenyl)-, -N-methyl-N-(4-chlorphenyl)-amid, -N-methyl-N-(3-nitro-6-methyl-phenyl)-amid, -N-äthyl-anilid, -N-äthyl-N-(2-nitro-phenyl)-, -N-äthyl-N-(3-nitro-phenyl)-, -N-äthyl-N-(4-nitro-phenyl)-amid, -N-äthyl-N-(2-chlor-phenyl)-, -N-äthyl-N-(3-chlorphenyl)-, -N-äthyl-N-(4-chlor-phenyl)-amid, -N-äthyl-N-(3-nitro-6-methyl-phenyl)-amid, -N-propyl-anilid, -N-propyl-N-(2-nitro-phenyl)-, -N-propyl-N-(3-nitro-phenyl)-, -N-propyl-N-(4-nitro-phenyl)-amid, -N-propyl-N-(2-chlor-phenyl)-, -N-propyl-N-(3-chlor-phenyl)-, -N-propyl-N-(4-chlor-phenyl)-amid, -N-propyl-N-(2-methyl-phenyl)-, -N-propyl-N-(3-methyl-phenyl)-, -N-propyl-N-(4-methylphenyl)-amid, -N-propyl-N-(3-nitro-6-methyl-phenyl)-amid, -N-butyl-anilid, -N-butyl-N-(2-nitro-phenyl)-, -N-butyl-N-(3-nitro-phenyl)-, -N-butyl-N-(4-nitrophenyl)-amid, -N-butyl-N-(2-chlor-phenyl)-, -N-butyl-N-(3-chlor-phenyl)-, -N-butyl-N-(4-chlor-phenyl)-amid, -N-butyl-N-(2-methyl-phenyl)-, -N-butyl-N-(3-methyl-phenyl)-, -N-butyl-N-(4-methyl-phenyl)-amid, -N-butyl-N-(3-nitro-6-methyl-phenyl)-amid, -N-iso-butyl-anilid, -N-iso-butyl-N-(2-nitro-phenyl)-, -N-iso-butyl-N-(3-nitro-phenyl)-, -N-iso-butyl-N-(4-nitro-phenyl)-amid, -N-iso-butyl-N-(2-chlor-phenyl)-, -N-iso-butyl-N-(3-chlor-phenyl)-, -N-isobutyl-N-(4-chlor-phenyl)-amid, -N-iso-butyl-N-(2-methylphenyl)-, -N-iso-butyl-N-(3-methyl-phenyl)-, -N-iso-butyl-N-(4-methyl-phenyl)-amid, -N-iso-butyl-N-(3-nitro-6-methyl-phenyl)-amid, -naphth(1)ylamid, -naphth(2)ylamid, -N-methyl-N-naphth(1)ylamid, -N-methyl-N-naphth(2)yl-amid, -N-äthyl-N-naphth(1)ylamid, -N-äthyl-N-naphth(2)ylamid, -N-n-propyl-N-naphth(2)ylamid, -N-iso-propyl-N-naphth(2)ylamid, -N-n-butyl-N-naphth(2)ylamid, -N-isobutyl-N-naphth(2)ylamid, -benzylamid, -dibenzylamid, -N-methyl-N-benzylamid, -N-äthyl-N-benzylamid, -N-propyl-N-benzylamid, -N-butyl-N-benzylamid, -pyrrolidid, -2-methyl-pyrrolidid, -morpholid, -piperidid, -2-methyl-piperidid, -4-methyl-piperidid, -2,4-dimethyl-piperidid, -2,4,6-trimethyl-piperidid, -2-äthyl-piperidid, -4-äthylpiperidid, -2-diäthyl-piperidid, -2,4,6-triäthyl-piperidid, -2-methyl-4-äthyl-piperidid, -2-äthyl-4-methyl-piperidid, -2-methyl-5-äthyl-piperidid, -2-äthyl-5-methyl-piperidid, -2-methyl-6-äthyl-piperidid, -1,2,3,4-tetrahydroindolid, -2-methyl-1,2,3,4-tetrahydroindolid, -perhydroindolid, -2-methyl-perhydroindolid, -2,2-dimethyl-perhydroindolid, -1,2,3,4-tetrahydrochinolid, -2-methyl-1,2,3,4-tetrahydrochinolid, -perhydrochinolid, -2-methyl-perhydrochinolid, -4-methyl-perhydrochinolid, -1,2,3,4-tetrahydroiso-chinolid und -perhydroisochinolid.

Die α-Halogencarbonsäureamide der Formel (II) sind bekannt oder können analog bekannten Verfahren hergestellt werden, beispielweise durch Umsetzung von α-Halogencarbonsäurehalogeniden wie z. B. Chloracetylchlorid mit Ammoniak bzw. primären oder sekundären Aminen, gegebenenfalls in Gegenwart eines Säureakzeptors wie z. B. Kaliumhydroxid (vergleiche J. Agric. Food Chem. 4 (1956), 518—522).

Die als Reagentien in der ersten Verfahrensstufe zu verwendenden Alkali- oder Erdalkaliacetate sind bekannt. Als Beispiele seien Natrium- und Kaliumacetat genannt.

Als Katalysatoren werden in der ersten Verfahrensstufe quartäre Ammoniumsalze verwendet. Als solche kommen Tetraalkylammoniumsalze in Frage, wobei die Alkylreste unabhängig voneinander 1 bis 4 Kohlenstoffatome enthalten können. Weiter kommen Trialkyl-aralkylammoniumsalze mit 1 bis 4 Kohlenstoffatomen je Alkylrest, 1 oder 2 Kohlenstoffatomen im Aralkyl-alkylteil und 6 oder 10 Kohlenstoffatomen im Aralkyl-arylteil als Katalysatoren in Betracht.

Als Beispiele seien genannt:

Tetra-n-butylammoniumbromid und Benzyltrimethylammoniumchlorid.

In der zweiten Verfahrensstufe werden als Katalysatoren Alkali- oder Erdalkalihydroxide bzw. Alkali- oder Erdalkalicarbonate verwendet.

Als Beispiele hierfür seien genannt:

Natriumhydroxid, Kaliumhydroxid, Calciumhydroxid, Natriumcarbonat und Kaliumcarbonat.

Die in der zweiten Verfahrensstufe als Reagentien und gleichzeitig als Verdünnungsmittel zu verwendenden Alkohole sind durch Formel (IV) definiert.

Vorzugsweise steht darin $R^4$ für geradkettiges oder verzweigtes Alkyl mit 1 bis 4 Kohlenstoffatomen. Als Beispiele seien Methanol, Äthanol, n- und iso-Propanol genannt.

Die erste Verfahrensstufe wird unter Verwendung geeigneter Lösungs- oder Verdünnungsmittel durchgeführt. Als solche kommen praktisch alle inerten organischen Solventien in Frage. Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Äthylenchlorid, Chlorbenzol und o-Dichlorbenzol; Äther wie Diäthyl- und Dibutyläther, Tetrahydrofuran und Dioxan; Ketone wie Aceton, Methyläthyl-, Methylisopropyl- und Methylisobutylketon sowie Nitrile wie Acetonitril und Propionitril.

Die Reaktionstemperaturen liegen für Stufe 1 im allgemeinen zwischen 20 und 200° C, vorzugsweise zwischen 50 und 150° C, für Stufe 2 zwischen 20 und 150° C, vorzugsweise zwischen 50 und 120° C. Beide Stufen des erfindungsgemäßen Verfahrens werden im allgemeinen bei Normaldruck durchgeführt.

Bei der Durchführung des erfindungsgemäßen Verfahrens setzt man in der ersten Verfahrensstufe auf 1 Mol α-Halogencarbonsäureamid (II) 0,9 bis 1,5 Mol, vorzugsweise 0,95 bis 1,2 Mol Acetat (wasserfrei) ein und verwendet 1 bis 20 mMol, vorzugsweise 2 bis 15 mMol Ammoniumsalz-Katalysator. Die Komponenten werden im allgemeinen in einem der angegebenen Verdünnungsmittel einige Stunden auf die erforderliche Reaktionstemperatur erhitzt, abgekühlt, und nach Filtration wird das Verdünnungsmittel abdestilliert. Die hierbei im Rückstand verbleibenden α-Acet-oxycarbonsäureamide werden zur Durchführung der zweiten Verfahrensstufe in 2 bis 5 Moläquivalenten eines der oben angegebenen Alkohole einige Stunden in Gegenwart von 1 bis 100 mMol, vorzugsweise 5 bis 50 mMol Hydroxid oder Carbonat erhitzt. Nach Abdestillieren des Alkoholüberschusses und des als Koppelprodukt gebildeten Esters erhält man im Rückstand die α-Hydroxy-carbonsäureamide, welche gegebenenfalls durch Umkristallisation gereinigt werden. Im allgemeinen fallen die α-Hydroxycarbonsäureamide nach dem erfindungsgemäßen Verfahren in hoher Reinheit an und können ohne weitere Reinigungsprozesse weiter verwendet werden.

Die erfindungsgemäß herstellbaren $\alpha$-Hydroxy-carbonsäureamide können als Zwischenprodukte zur Herstellung von Herbiziden verwendet werden (vgl. z. B. US-PS 3 399 988, DE-OS 2 201 432 und DE-OS 2 647 568).

## Herstellungsbeispiele

### Beispiel 1:

(a) Zwischenprodukt:

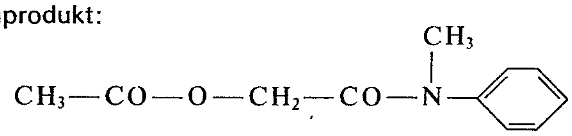

Eine Suspension aus 183,5 g (1 Mol) Chloressigsäure-N-methylanilid, 82 g (1 Mol) wasserfreiem Natriumacetat, 0,5 g Benzyltrimethylammoniumchlorid als Katalysator und 320 ml Toluol wird 4 Stunden auf 115—120° C erhitzt und dann auf Raumtemperatur abgekühlt. Der Ansatz wird abgesaugt und der Rückstand mit kaltem Toluol nachgewaschen. Aus der toluolischen Lösung werden nach Abdestillieren des Lösungsmittels und eindampfen des Rückstandes im Dampfstrahlvakuum bei einer Badtemperatur von 80—85° C 207 g $\alpha$-Acetoxy-essigsäure-N-methylanilid erhalten, das beim Stehen kristallisiert — GC*). = 98%ig,
Schmelzpunkt 54—56° C; Ausbeute 99% der Theorie
(ohne Katalysator-Zusatz wird nur eine Ausbeute von 23,8% der Theorie erreicht).

(b) Endprodukt:

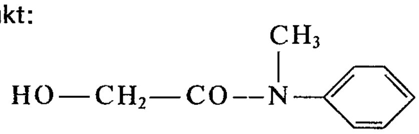

Das Reaktionsgemisch aus 211,2 g (1 Mol) $\alpha$-Acetoxyessigsäure-N-methylanilid (98%ig), 0,2 g Natriumhydroxid und 160 g Methanol wird 4 Stunden unter Rückfluß erhitzt. Das Gemisch aus Methanol und Methylacetat wird abdestilliert. Der flüssige Destillationsrückstand [170 g Ausbeute an Hydroxy-essigsäure-N-methylanilid, quantitativ, GC*: 98%, Schmelzpunkt: 52—53° C] erstarrt beim Abkühlen.

### Beispiel 2:

(a) Zwischenprodukt:

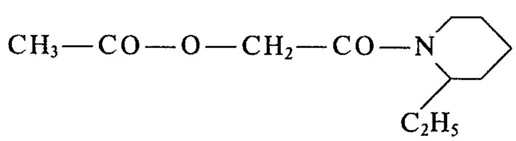

Eine Suspension aus 32,8 g (0,169 Mol) Chloressigsäure-2-äthylpiperidid (97,8%ig), 14,4 g (0,175 Mol) Natriumacetat (wasserfrei), 0,5 g Benzyltrimethylammoniumchlorid als Katalysator und 50 ml Toluol wird 5 Stunden auf 115—120° C erhitzt. Nach Abkühlen des Reaktionsansatzes auf Raumtemperatur wird der anorganische Rückstand abgesaugt und mit Toluol gewaschen. Aus der toluolischen Lösung werden nach Abdestillieren des Lösungsmittels und Eindampfen des Rückstandes bei einer Badtemperatur von 80—85° C im Dampfstrahlvakuum 36,1 g flüssiges $\alpha$-Acetoxyessigsäure-2-äthylpiperidid erhalten in einer Ausbeute von 96,7% der Theorie. GC: 96,4%ig.
(ohne Katalysator-Zusatz wird nur eine Ausbeute von 23% der Theorie erreicht.)

*) GC = gaschromatographische Analyse

(b) Endprodukt:

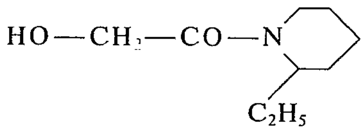

Das Reaktionsgemisch aus 22 g (0,1 Mol) α-Acetoxyessigsäure-2-äthylpiperidid (97%ig), 0,2 g Kaliumhydroxid und 50 ml Methanol wird 5 Stunden unter Rückfluß erhitzt. Das Gemisch aus Methanol und Methylacetat wird abdestilliert. Es wird ein flüssiger Destillationsrückstand erhalten an Hydroxy-essigsäure-2-äthyl-piperidid in einer Ausbeute von 97,3% der Theorie. GC: 97,3%.

Analog Beispiel 1 oder 2 können beispielsweise die in den nachstehenden Tabellen aufgeführten Verbindungen der Formel (I) bzw. (III) hergestellt werden:

Tabelle I:

Verbindungen der Formel (III) 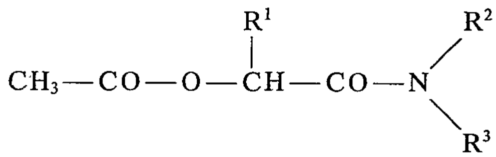

| Beispiel Nr. | $R^1$ | 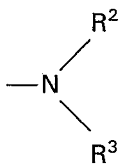 | Ausbeute (% der Theorie) | Schmelzpunkt (°C) Brechungsindex: |
|---|---|---|---|---|
| 3 (a) | H | 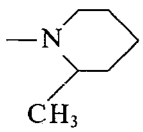 | 99 | |
| 4 (a) | H | 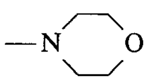 | | |
| 5 (a) | H | $—N(CH_2—CH_2—OCH_3)_2$ | 91 | $n_D^{25}$ 1,4880 |
| 6 (a) | H | 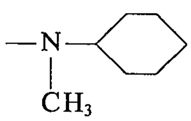 | 100 | $n_D^{25}$ 1,4816 |
| 7 (a) | H | 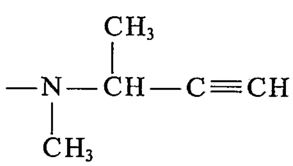 | 98 | $n_D^{25}$ 1,4718 |
| 8 (a) | H | 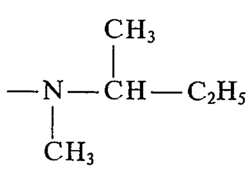 | | |
| 9 (a) | H | 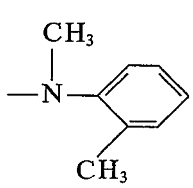 | | |

Fortsetzung

| Beispiel Nr. | $R^1$ | 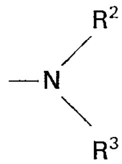 | Ausbeute (% der Theorie) | Schmelzpunkt (°C) Brechungsindex: |
|---|---|---|---|---|
| 10 (a) | H | 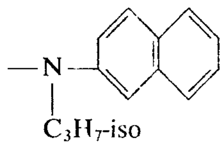 | 91 | $n_D^{25}$ 1,4760 |
| 11 (a) | H | 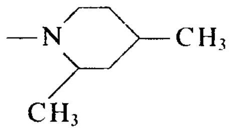 | 80 | $n_D^{24}$ 1,4997 |
| 12 (a) | H | 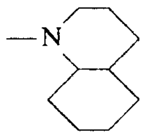 | | |
| 13 (a) | H | 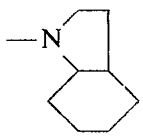 | | |
| 14 (a) | H | 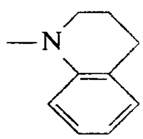 | 69 | 97° |
| 15 (a) | H | 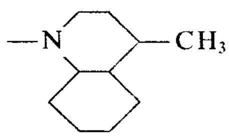 | 87 | $n_D^{25}$ 1,4932 |
| 16 (a) | H | $-N(CH_2-CH=CH_2)_2$ | 100 | $n_D^{25}$ 1,4748 |
| 17 (a) | H | $-N(C_2H_5)_2$ | 80 | $n_D^{20}$ 1,4520 |

Tabelle II:

Verbindungen der Formel (I) 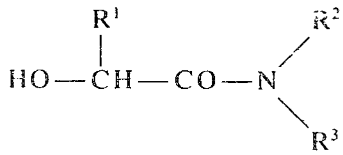

| Beispiel Nr. | $R^1$ | 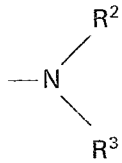 | Ausbeute (% der Theorie) | Schmelzpunkt (°C) Brechungsindex: |
|---|---|---|---|---|
| 3 (b) | H | 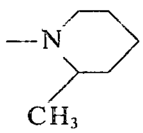 | 100 | 36° |
| 4 (b) | H | 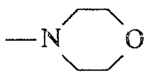 | | |
| 5 (b) | H | $—N(CH_2—CH_2—OCH_3)_2$ | 100 | $n_D^{25}$ 1,4662 |
| 6 (b) | H | 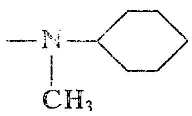 | 100 | 83° |
| 7 (b) | H | 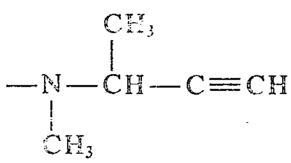 | 97,4 | $n_D^{25}$ 1,4859 |
| 8 (b) | H | 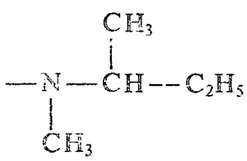 | | |
| 9 (b) | H | 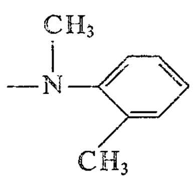 | | |
| 10 (b) | H | 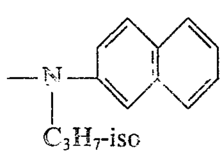 | | |
| 11 (b) | H | 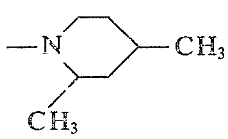 | 60 | $n_D^{23}$ 1,4816 |

Fortsetzung

| Beispiel Nr. | $R^1$ | 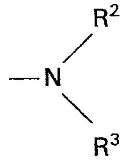 | Ausbeute (% der Theorie) | Schmelzpunkt (°C) Brechungsindex: |
|---|---|---|---|---|
| 12 (b) | H | 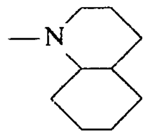 | 65 | 55°C |
| 13 (b) | H | 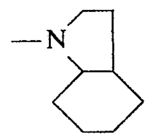 | 70 | $n_D^{23}$ 1,5076 |
| 14 (b) | H | 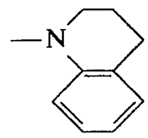 | 61 | 80° |
| 15 (b) | H | 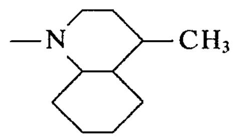 | 63 | |
| 16 (b) | H | $-N(CH_2-CH=CH_2)_2$ | | |
| 17 (b) | H | $-N(C_2H_5)_2$ | 94 | Siedepunkt: 80°C/5,3 mbar |

**Patentansprüche**

1. Verfahren zur Herstellung von α-Hydroxycarbonsäureamiden der Formel

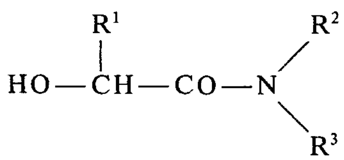 (I)

in welcher

$R^1$ für Wasserstoff oder Alkyl steht,

$R^2$ und $R^3$ gleich oder verschieden sind und einzeln für Wasserstoff, jeweils gegebenenfalls durch Cyano-, Alkoxy- oder Alkylthioreste substituiertes Alkyl, Alkenyl, Alkinyl oder Cycloalkyl, jeweils gegebenenfalls durch Halogenatome, Alkyl-, Nitro- und/oder Cyanoreste substituiertes Aralkyl oder Aryl oder einen stickstoffhaltigen heterocyclischen Rest stehen oder zusammen mit dem Stickstoffatom, an das sie gebunden sind, einen gegebenenfalls durch Alkylgruppen substituierten, gegebenenfalls teilweise ungesättigten und gegebenenfalls benzanellierten Mono- oder Bicyclus bilden, der gegebenenfalls weitere Heteroatome enthält,

durch Umsetzung von α-Halogencarbonsäureamiden mit Acetaten zu α-Acetoxycarbonsäureamiden und Entacylierung dieser Zwischenprodukte, dadurch gekennzeichnet, daß man in erster Stufe α-Halogencarbonsäureamide der Formel

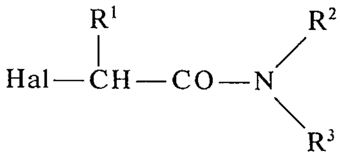

(II)

in welcher

$R^1$, $R^2$ und $R^3$ die oben angegbene Bedeutung haben und
Hal für Chlor oder Brom steht,

in Gegenwart eines quartären Ammoniumsalzes mit einem in einem inerten organischen Solvens suspendierten Alkali- oder Erdalkaliacetat bei Temperaturen zwischen 20 und 200° C umsetzt und in zweiter Stufe die auf diese Weise hergestellten α-Acetoxycarbonsäureamide der Formel

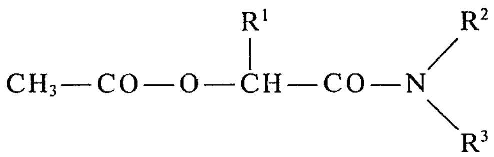

(III)

in welcher

$R^1$, $R^2$ und $R^3$ die oben angegebene Bedeutung haben,

in an sich bekannter Weise durch Umsetzung mit einem Alkohol der Formel

$$R^4—OH \qquad (IV)$$

in welcher

$R^4$ für Alkyl steht,

in Gegenwart katalytischer Mengen eines Alkali- oder Erdalkalihydroxides oder eines Alkali- oder Erdalkalicarbonates bei Temperaturen zwischen 20 und 150° C entacycliert.

2. Verfahren nach Anspruch 1, dadurch gekennzeichnet, daß man die erste Reaktionsstufe bei Temperaturen zwischen 50 und 150° C durchführt.

3. Verfahren nach Anspruch 1, dadurch gekennzeichnet, daß man die zweite Reaktionsstufe bei Temperaturen zwischen 50 und 120° C durchführt.

4. Verfahren nach Anspruch 1, dadurch gekennzeichnet, daß als quartäres Ammoniumsalz ein Tetraalkylammoniumsalz mit 1 bis 4 Kohlenstoffatomen je Alkylrest oder ein Trialkyl-aralkylammoniumsalz mit 1 bis 4 Kohlenstoffatomen je Alkylrest, 1 oder 2 Kohlenstoffatomen im Aralkyl-alkylteil und 6 oder 10 Kohlenstoffatomen im Aralkyl-arylteil, eingesetzt wird.

5. Verfahren nach Anspruch 4, dadurch gekennzeichnet, daß als quartäres Ammoniumsalz Tetra-n-butylammoniumbromid oder Benzyltrimethylammoniumchlorid eingesetzt wird.

6. Verfahren nach Anspruch 1, dadurch gekennzeichnet, daß man in der ersten Reaktionsstufe auf 1 Mol α-Halogencarbonsäureamid (II) 0,9 bis 1,5 Mol, vorzugsweise 0,95 bis 1,2 Mol Acetat (wasserfrei) und 1 bis 20 mMol, vorzugsweise 2 bis 15 mMol des quartären Ammoniumsalzes einsetzt.

7. Verfahren nach Anspruch 1, dadurch gekennzeichnet, daß man in der zweiten Reaktionsstufe auf das aus 1 Mol α-Halogencarbonsäureamid (II) hergestellte α-Acetoxycarbonsäureamid (III) 2 bis 5 Moläquivalente Alkohol (IV) und 1 bis 100 mMol, vorzugsweise 5 bis 50 mMol eines Alkali- oder Erdalkalihydroxides oder eines Alkali- oder Erdalkalicarbonates einsetzt.

8. Verfahren zur Herstellung von α-Acetoxycarbonsäureamiden der Formel

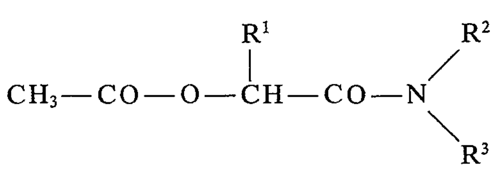

(III)

in welcher

$R^1$, $R^2$ und $R^3$ die in Anspruch 1 angegebene Bedeutung haben,

durch Umsetzung von α-Halogencarbonsäureamiden mit Acetaten, dadurch gekennzeichnet, daß man

α-Halogencarbonsäureamide der Formel

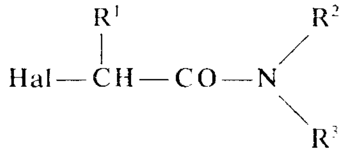

(II)

in welcher

$R^1$, $R^2$ und $R^3$ die oben angegebene Bedeutung haben und
Hal für Chlor oder Brom steht,

in Gegenwart eines quartären Ammoniumsalzes mit einem in einem inerten organischen Solvens suspendierten Alkali- oder Erdalkaliacetat bei Temperaturen zwischen 20 und 200° C umsetzt.

**Claims**

1. Process for the preparation of α-hydroxycarboxylic acid amides of the formula

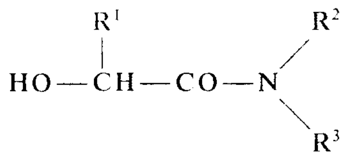

(I)

in which

$R^1$ represents hydrogen or alkyl,
$R^2$ and $R^3$ are identical or different and individually represent hydrogen, or alkyl, alkenyl, alkynyl or cycloalkyl, in each case optionally substituted by cyano, alkoxy or alkylthio radicals, or aralkyl or aryl, in each case optionally substituted by halogen atoms, alkyl, nitro and/or cyano radicals, or a nitrogen-containing heterocyclic radical or, together with the nitrogen atom to which they are bonded, form a mono- or bi-cyclic ring which is optionally substituted by alkyl groups, optionally partially unsaturated and optionally benzo-fused, and which optionally contains further hetero-atoms,

by reacting α-halogenocarboxylic acid amides with acetates to give α-acetoxycarboxylic acid amides and deacylating these intermediate products, characterised in that, in a first stage, α-halogenocarboxylic acid amides of the formula

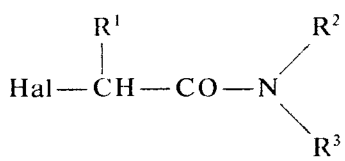

(II)

in which

$R^1$, $R^2$ and $R^3$ have the meaning indicated above and
Hal represents chlorine or bromine,

are reacted with an alkali metal acetate or alkaline earth metal acetate suspended in an inert organic solvent, in the presence of a quaternary ammonium salt, at temperatures between 20 and 200° C and, in a second stage, the α-acetoxycarboxylic amides prepared in this manner, of the formula

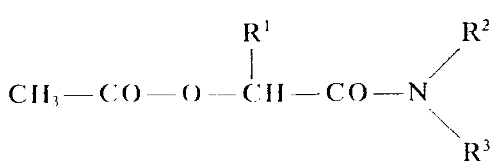

(III)

in which

$R^1$, $R^2$ and $R^3$ have the meaning indicated above, are deacylated in a manner known per se by reaction with an alcohol of the formula

$$R^4—OH \qquad (IV)$$

in which

$R^4$ represents alkyl,
in the presence of catalytic amounts of an alkali metal hydroxide or alkaline earth metal hydroxide or of an alkali metal carbonate or alkaline earth metal carbonate, at temperatures between 20 and 150° C.

2. Process according to claim 1, characterised in that the first reaction stage is carried out at temperatures between 50 and 150° C.

3. Process according to claim 1, characterised in that the second reaction stage is carried out at temperatures between 50 and 120° C.

4. Process according to claim 1, characterised in that a tetraalkylammonium salt with 1 to 4 carbon atoms per alkyl radical or a trialkyl-aralkylammonium salt with 1 to 4 carbon atoms per alkyl radical, 1 or 2 carbon atoms in the aralkyl-alkyl part and 6 or 10 carbon atoms in the aralkylaryl part is employed as the quaternary ammonium salt.

5. Process according to claim 4, characterised in that tetra-n-butylammonium bromide or benzyltrimethylammonium chloride is employed as the quaternary ammonium salt.

6. Process according to claim 1, characterised in that, in the first reaction stage, 0.9 to 1.5 mols, preferably 0.95 to 1.2 mols, of acetate (anhydrous) and 1 to 20 mmols, preferably 2 to 15 mmols, of the quaternary ammonium salt are employed per 1 mol of α-halogenocarboxylic acid amide (II).

7. Process according to claim 1, characterised in that, in the second reaction stage, 2 to 5 mol equivalents of alcohol (IV) and 1 to 100 mmols, preferably 5 to 50 mmols, of an alkali metal hydroxide or alkaline earth metal hydroxide or of an alkali metal carbonate or alkaline earth metal carbonate are employed with the α-acetoxycarboxylic acid amide (III) prepared from 1 mol of α-halogenocarboxylic acid amide (II).

8. Process for the preparation of α-acetoxycarboxylic acid amides of the formula

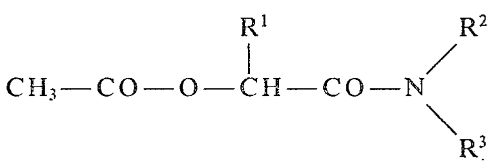

(III)

in which

$R^1$, $R^2$ and $R^3$ have the meaning indicated in claim 1, by reacting α-halogenocarboxylic acid amides with acetates, characterised in that α-halogenocarboxylic acid amides of the formula

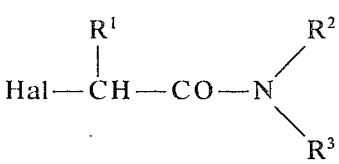

(II)

in which

$R^1$, $R^2$ and $R^3$ have the meaning indicated above and
Hal represents chlorine or bromine,

are reacted with an alkali metal acetate or alkaline earth metal acetate suspended in an inert organic solvent, in the presence of a quaternary ammonium salt, at temperatures between 20 and 200° C.

**Revendications**

1. Procédé de production d'amides d'acides α-hydroxycarboxyliques de formule

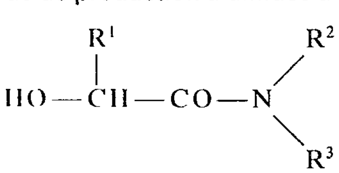

(I)

dans laquelle

$R^1$ est l'hydrogène ou un groupe alkyle,

$R^2$ et $R^3$ sont égaux ou différents et représentent individuellement de l'hydrogène, un groupe alkyle, alcényle, alcynyle ou cyclo-alkyle dont chacun est éventuellement substitué par des restes cyano, alkoxy ou alkylthio, un groupe aralkyle ou aryle dont chacun est éventuellement substitué par des atomes d'halogènes, des restes alkyle, nitro et/ou cyano ou un reste hétérocyclique contenant de l'azote, ou forment conjointement avec l'atome d'azote auquel ils sont liés, un cycle simple ou double éventuellement substitué par des groupes alkyle, éventuellement en partie non saturé et, le cas échéant, condensé à du benzène, qui comporte éventuellement d'autres hétéro-atomes,

par réaction d'amides d'acides $\alpha$-halogénocarboxyliques avec des acétates pour former des amides d'acides $\alpha$-acétoxycarboxyliques, et désacylation de ces produits intermédiaires, caractérisé en ce qu'on fait réagir dans une première étape des amides d'acides $\alpha$-halogénocarboxyliques de formule

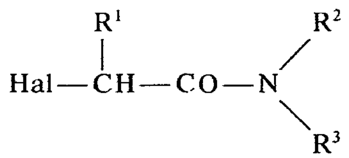

(II)

dans laquelle

$R^1$, $R^2$ et $R^3$ ont la définition indiquée ci-dessus et

Hal désigne du chlore ou du brome,

en présence d'un sel d'ammonium quaternaire, avec un acétate alcalin ou alcalino-terreux en suspension dans un solvant organique inerte, à des températures comprises entre 20 et 200° C, et on désacyle dans une seconde étape les amides d'acides $\alpha$-acétoxycarboxyliques préparés de cette façon, de formule

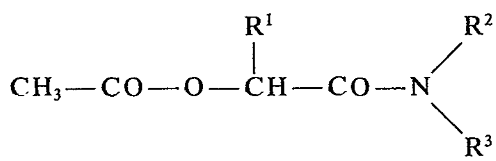

(III)

dans laquelle

$R^1$, $R^2$ et $R^3$ ont la définition indiquée ci-dessus, d'une manière connue par réaction avec un alcool de formule

$$R^4\text{—OH} \qquad \text{(IV)}$$

dans laquelle

$R^4$ est un groupe alkyle,

en présence de quantités catalytiques d'un hydroxyde de métal alcalin ou alcalino-terreux ou d'un carbonate de métal alcalin ou alcalino-terreux, à des températures comprises entre 20 et 150° C.

2. Procédé suivant la revendication 1, caractérisé en ce qu'on conduit la première étape réactionnelle à des températures comprises entre 50 et 150° C.

3. Procédé suivant la revendication 1, caractérisé en ce qu'on conduit la seconde étape réactionnelle à des températures comprises entre 50 et 120° C.

4. Procédé suivant la revendication 1, caractérisé en ce qu'on utilise comme sel d'ammonium quaternaire un sel de tétra-alkylammonium ayant 1 à 4 atomes de carbone par reste alkyle ou un sel de trialkyl-aralkylammonium ayant 1 à 4 atomes de carbone par reste alkyle, 1 ou 2 atomes de carbone dans la partie alkylique d'aralkyle et 6 à 10 atomes de carbone dans la partie arylique d'aralkyle.

5. Procédé suivant la revendication 4, caractérisé en ce qu'on utilise comme sel d'ammonium quaternaire le bromure de tétra-n-butylammonium ou le chlorure de benzyltriméthylammonium.

6. Procédé suivant la revendication 1, caractérisé en ce qu'on utilise dans la première étape réactionnelle, par mole d'amide d'acide $\alpha$-halogénocarboxylique (II), 0,9 à 1,5 mole, de préférence 0,95 à 1,2 mole, d'acétate (anhydre) et 1 à 20 mmoles, de préférence 2 à 15 mmoles, du sel d'ammonium quaternaire.

7. Procédé suivant la revendication 1, caractérisé en ce qu'on utilise dans la seconde étape réactionnelle, sur la base ce l'amide d'acide $\alpha$-acétoxycarboxylique (III) préparé à partir d'une mole

d'amide d'acide α-halogénocarboxylique (II), 2 à 5 équivalents molaires d'alcool (IV) et 1 à 100 mmoles, de préférence 5 à 50 mmoles, d'un hydroxyde alcalin ou alcalino-terreux ou d'un carbonate alcalin ou alcalino-terreux.

8. Procédé de production d'amides d'acides α-acétoxycarboxyliques de formule

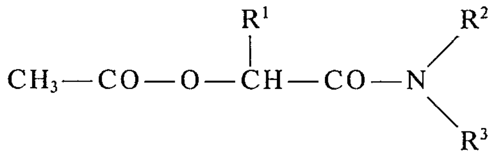

(III)

dans laquelle

$R^1$, $R^2$ et $R^3$ ont la définition indiquée dans la revendication 1,

par réaction d'amides d'acides α-halogénocarboxyliques avec des acétates, caractérisé en ce qu'on fait réagir des amides d'acides α-halogénocarboxyliques de formule

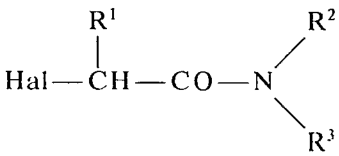

(II)

dans laquelle

$R^1$, $R^2$ et $R^3$ ont la définition indiquée ci-dessus et
Hal désigne du chlore ou du brome,

en présence d'un sel d'ammonium quaternaire avec un acétate alcalin ou alcalino-terreux en suspension dans un solvant organique inerte, à des températures comprises entre 20 et 200° C.